# EUROPEAN PATENT APPLICATION

(11) **EP 3 748 017 A1**
(43) Date of publication of application: **09.12.2020**
(21) Application number: 19179201.9
(22) Date of filing: 07.06.2019
(51) Int. Cl.: C12Q 1/6883

(54) **METHYLATION MARKERS FOR PREDICTING SENSITIVITY TO TREATMENT WITH ANTIBODY BASED THERAPY**

(71) Applicant: Academisch Medisch Centrum, 1105 AZ Amsterdam (NL)
(72) Inventor: The designation of the inventor has not yet been filed
(74) Representative: de Pauw, Elmar Sebastian David

(57) **Abstract**

The present invention provides a method of determining or predicting the sensitivity of a subject to an anti-inflammatory treatment using a monoclonal antibody targeting tumor necrosis factor α or integrin α4β7, wherein said method is based on determining the methylation level in at least one locus in the DNA of a biological sample of a subject.

## Description

### TECHNICAL FIELD OF THE INVENTION

The present invention relates in general to the use of the methylation status of particular loci positioned on genomic DNA and their potential to predict drug resistance/susceptibility to the treatment of Crohn's disease (CD) and ulcerative colitis (UC), referred to as inflammatory bowel diseases (IBD). More specifically, the invention relates to the DNA methylation status as biomarkers for predicting the efficacy of treatment using infliximab, vedolizumab and adalimumab in the treatment of CD.

### BACKGROUND OF THE INVENTION

CD is an IBD with an unknown etiology(1). Based on advancing knowledge of the pathophysiology new therapeutic strategies have been developed that target the pathologic pathways in CD(2)(3). For over twenty years antibodies to TNF-α (infliximab/adalimumab (ADA) have been used and recently also antibodies that target leukocyte trafficking (vedolizumab (VEDO) and other cytokines (IL-12/IL-23) demonstrated efficacy for treating CD(4)(5)(6)(7)(8). Biomarker discovery for CD based on the personalized genotype, clinical information and immunological reactivity could facilitate the stratification of patients to optimally tailored choice for therapeutic approaches. Current clinical factors confer little power for predicting response and, in current practice, an unsatisfactory 'trial and error' approach governs therapeutic decisions. A recent review of the decision-making process in IBD advocates a tailored individual approach since less than half of the patients benefit from anti-TNF therapy(9). Similar percentages are reported for other biological therapies(10)(11)(12). Despite the fact that many IBD patients respond to the aforementioned biologicals, a sizeable population either does not respond or becomes non-responsive (18-21). While dose escalation regains responsiveness for some patients, many remain unresponsive (22-24). To this end, a strong necessity exists to predict whether and understand why a patient does not respond to a particular drug treatment.

Candidate protein, gene and unbiased genome-wide investigations have sought to identify biomarkers that predict who will respond to therapy (13). So far, there are no peripheral biomarkers available that can predict who will respond to therapy with biological drugs, i.e. current biomarkers are in the daily practice only used to monitor the response during the course of the administration of the evaluated therapy and in specific tissues. The latter needs an invasive procedure such as endoscopy of the gastrointestinal tract. Therefore, there is a need in the art to for biomarkers which predict the sensitivity of a subject to an anti-inflammatory treatment using a antibodies targeting TNF-α or integrin α4β7.

### SUMMARY OF THE INVENTION

This invention is based on the identification of differential DNA-methylation in the DNA obtained from whole blood, that predict a response for a treatment of IBD using Infliximab; a monoclonal antibody against TNF-α, which prevents TNF-α receptor binding, Adalimumab; a monoclonal antibody against TNF-α - which prevents TNF-α receptor binding and binds to membrane-bound TNF, and Vedolizumab; a monoclonal antibody against integrin α4β7 (LPAM-1, lymphocyte Peyer's patch adhesion molecule 1, or (3).

While several response associated genetic, transcriptomic and protein markers have been documented in blood and intestinal biopsies (25,26), no evidence exists that such markers can be found at the level of DNA methylation.

The invention therefore provides a method of determining or predicting the sensitivity of a subject to an anti-inflammatory treatment against IBD, using an antibody inhibiting binding of TNF-α to its receptor or an antibody blocking integrin α4β7, wherein said method is based on determining the methylation status in at least one differentially methylated region (DMR) in the DNA of a biological sample of a subject.

As disclosed herein, the inventors investigated the association of peripheral blood DNA methylation and response to vedolizumab, adalimumab, or infliximab treatment in CD patients.

The inventors found 38 differentially methylated regions (DMRs) for infliximab, 9 DMRs for vedoluzimab and 11 DMRs for adalumimab (see Table 1). Therefore, in a preferred embodiment, said method comprises determining, in at least one DMR of said DNA, the methylation status at one or more CpGs located within one or more of the nucleotide sequences comprised in one or more of the respective DMRs of the present invention independently selected from the group consisting of:
cg11261447 (SEQ ID NO:1), cg07460376 (SEQ ID NO:2), cg05155812 (SEQ ID NO:3),
cg08332658 (SEQ ID NO:4), cg26175789 (SEQ ID NO:5), cg01615818 (SEQ ID NO:6),
cg09255157 (SEQ ID NO:7), cg14234990 (SEQ ID NO:8), cg04850148 (SEQ ID NO:9),
cg00356897 (SEQ ID NO:10), cg01847718 (SEQ ID NO:11), cg01257975 (SEQ ID NO:12),
cg09163005 (SEQ ID NO:13), cg12806797 (SEQ ID NO:14), cg05014687 (SEQ ID NO:15),
cg07112131 (SEQ ID NO:16), cg16322792 (SEQ ID NO:17), cg09169455 (SEQ ID NO:18),
cg24491749 (SEQ ID NO:19), cg03856973 (SEQ ID NO:20), cg20649823 (SEQ ID NO:21),
cg23831005 (SEQ ID NO:22), cg07068406 (SEQ ID NO:23), cg05871254 (SEQ ID NO:24),
cg17758362 (SEQ ID NO:25), cg06524124 (SEQ ID NO:26), cg26127836 (SEQ ID NO:27),
cg01249606 (SEQ ID NO:28), cg12754571 (SEQ ID NO:29), cg19736195 (SEQ ID NO:30),
cg23133460 (SEQ ID NO:31), cg23043514 (SEQ ID NO:32), cg06277607 (SEQ ID NO:33),
cg12418129 (SEQ ID NO:34), cg10564845 (SEQ ID NO:35), cg13959831 (SEQ ID NO:36),
cg21593676 (SEQ ID NO:37), cg02847897 (SEQ ID NO:38),
cg13679303 (SEQ ID NO:39), cg14598338 (SEQ ID NO:40), cg06031268 (SEQ ID NO:41),
cg20603222 (SEQ ID NO:42), cg26224354 (SEQ ID NO:43), cg06378142 (SEQ ID NO:44),
cg06406458 (SEQ ID NO:45), cg05622025 (SEQ ID NO:46), cg09715000 (SEQ ID NO:47), or,
cg26913836 (SEQ ID NO:48), cg15488109 (SEQ ID NO:49), cg25873494 (SEQ ID NO:50),
cg25123302 (SEQ ID NO:51), cg01379846 (SEQ ID NO:52), cg08944241 (SEQ ID NO:53),
cg04377463 (SEQ ID NO:54), cg03299990 (SEQ ID NO:55), cg00683245 (SEQ ID NO:56),
cg12793466 (SEQ ID NO:57), cg05894480 (SEQ ID NO:58)
or a nucleotide sequence present within about 2,000bp, more preferably within about 250, 200bp 5' or 3' thereof, or an allelic variant and/or complementary sequence of any of said nucleotide sequences.

In a preferred embodiment, said DMR is selected from the group consisting of:
SEQ ID NO:1-38, SEQ ID NO:1-37, SEQ ID NO:1-36, SEQ ID NO:1-35, SEQ ID NO:1-34, SEQ ID NO:1-33, SEQ ID NO:1-32, SEQ ID NO:1-31, SEQ ID NO:1-30, SEQ ID NO:1-29, SEQ ID NO:1-28, SEQ ID NO:1-27, SEQ ID NO:1-26, SEQ ID NO:1-25, SEQ ID NO:1-24, SEQ ID NO:1-23, SEQ ID NO:1-22, SEQ ID NO:1-21, SEQ ID NO:1-20, SEQ ID NO:1-19, SEQ ID NO:1-18, SEQ ID NO:1-17, SEQ ID NO:1-16, SEQ ID NO:1-15, SEQ ID NO:1-14, SEQ ID NO:1-13, SEQ ID NO:1-12, SEQ ID NO:1-11, SEQ ID NO:1-10, SEQ ID NO:1-9, SEQ ID NO:1-8, SEQ ID NO:1-7, , SEQ ID NO:1-6, SEQ ID NO:1-5, SEQ ID NO:1-4, SEQ ID NO:1-3, SEQ ID NO:1-2 or a nucleotide sequence present within about 2,000bp, more preferably within about 250, 200bp 5' or 3' thereof, or an allelic variant and/or complementary sequence of any of said nucleotide sequences. In another preferred embodiment, said DMR is selected from the group consisting of: SEQ ID NO:39-47, SEQ ID NO:39-46, SEQ ID NO:39-45, SEQ ID NO:39-44, SEQ ID NO:39-43, SEQ ID NO:39-42, SEQ ID NO:39-41, SEQ ID NO:39-40, or a nucleotide sequence present within about 2,000bp, more preferably within about 250, 200bp 5' or 3' thereof, or an allelic variant and/or complementary sequence of any of said nucleotide sequences.

In another preferred embodiment, said DMR is selected from the group consisting of:
SEQ ID NO:48-58, SEQ ID NO:48-57, SEQ ID NO:48-56, SEQ ID NO:48-55, SEQ ID NO:48-54, SEQ ID NO:48-53, SEQ ID NO:48-52, SEQ ID NO:48-51, SEQ ID NO:48-50, SEQ ID NO:48-49, or a nucleotide sequence present within about 2,000bp, more preferably within about 250, 200bp 5' or 3' thereof, or an allelic variant and/or complementary sequence of any of said nucleotide sequences.

In a preferred embodiment of the method according to the invention, a higher level of methylation of cg11261447, cg05155812, cg26175789, cg09255157, cg00356897, cg05014687, cg24491749, cg03856973, cg07068406, cg17758362, cg06524124, cg19736195, cg23043514, cg06277607, cg13959831, cg21593676c and cg02847897 in comparison to a control value is indicative of an increased sensitivity to a therapy using infliximab, and wherein said antibody is infliximab.

In another preferred embodiment of the method according to the invention, a lower level of methylation of cg07460376, cg08332658, cg01615818, cg14234990, cg04850148, cg01847718, cg01257975, cg09163005, cg12806797, cg07112131, cg16322792, cg09169455, cg20649823, cg23831005, cg05871254, cg26127836, cg01249606, cg12754571, cg23133460, cg12418129 and cg10564845 in comparison to a control value is indicative of an increased sensitivity to a therapy using infliximab, and wherein said antibody is infliximab.

In another preferred embodiment of the method according to the invention, a higher level of methylation of cg06031268, cg20603222, cg26224354, cg06378142, cg05622025, and cg09715000 in comparison to a control value is indicative of an increased sensitivity to a therapy using vedolizumab, and wherein said antibody is vedolizumab.

In another preferred embodiment of the method according to the invention, a lower level of methylation of cg13679303, cg14598338 and cg06406458 in comparison to a control value is indicative of an increased sensitivity to a therapy using vedolizumab, and wherein said antibody is vedolizumab.

In another preferred embodiment of the method according to the invention, a higher level of methylation of cg26913836, cg15488109, cg01379846, cg08944241, cg03299990 and cg00683245 in comparison to a control value is indicative of an increased sensitivity to a therapy using adalimumab, and wherein said antibody is adalimumab.

In another preferred embodiment of the method according to the invention, a lower level of methylation of cg25873494, cg25123302, cg04377463, cg12793466 and cg05894480 in comparison to a control value is indicative of an increased sensitivity to a therapy using adalimumab, and wherein said antibody is adalimumab.

Preferably, said IBD comprises CD.

In a preferred embodiment, said biological sample comprises white blood cells. Preferably, said methylation level is determined using DNA methylation array, preferably an Illumina BeadChip EPIC array.

The invention further provides Infliximab for use in the treatment of a subject who is sensitive to a treatment with infliximab as determined using the method according to the invention.

The invention further provides Vedolizumab for use in the treatment of a subject who is sensitive to a treatment with vedolizumab as determined using the method according to the invention.

The invention further provides Adalimumab for use in the treatment of a subject who is sensitive to a treatment with Adalimumab as determined using the method according to the invention.

The invention further provides the use of a DNA methylation array for the performing the method according to the invention, preferably an Illumina BeadChip EPIC array.

### DETAILED DESCRIPTION OF THE INVENTION

### Definitions

The term "biological sample" as used herein refers to any sample from an IBD patient for diagnostic, prognostic, or personalized medicinal uses and may be obtained from surgical samples, such as biopsies or fine needle aspirates, from paraffin-embedded tissues, from a fresh or frozen body fluid. Most preferably the sample contains nucleated blood cells. However, any other suitable biological samples (e.g. bodily fluids such as stool, etc...) in which the methylation status of a locus of interest can be determined are included within the scope of the invention.

By "methylation status" is meant the level of methylation of cytosine residues (found in CpG pairs) in the DNA sequence of interest. When used in reference to a CpG site, the methylation status may be methylated or unmethylated. When used in reference to a CpG island or to any stretch of residues, the methylation status refers to the level of methylation, which is the relative or absolute concentration of methylated C at the particular CpG island or stretch of residues in the DNA present in a biological sample.

The term "hypermethylation" as used herein refers to the average methylation status corresponding to an increased presence of 5-mCyt at one or a plurality of CpG dinucleotides within a DNA sequence of a test DNA sample, relative to the amount of 5-mCyt found at corresponding CpG dinucleotides from a DNA sample from a non-responder. Preferably, a responder is a subject who meets the following criteria at their follow-up endoscopy between 6-24 months after starting biological treatment (if no major medication changes during this period):
a)- ≥50% drop in the endoscopic severity score SES-CD score compared to baseline endoscopy, and
b)- ≥3 point drop in the clinical Harvey Bradshaw index (51) and/or
c)- shows signs of biological improvement based on either a CRP <5.0 mg/l or fecal calprotectin <250ug/g, and
d)- need no steroid treatment at response assessment.

A non- responder is preferably a subject who meets the following criteria at their follow-up endoscopy between 6-24 months after starting biological treatment (if no major medication changes during this period):
a)- <50% drop in SES-CD score compared to baseline endoscopy, and
b)- <3 point drop in Harvey Bradshaw index, and/or
c)- shows no signs of biological improvement based on either an unchanged or increased (delta 25%) CRP and/or fecal calprotectin >250ug/g, and/or
d)- is in need of a steroid treatment at response assessment.

The phrase "corresponding to" when used to describe positions or sites within nucleotide sequences, is used herein as it is understood in the art. As is well known in the art, two or more nucleotide sequences can be aligned using standard bioinformatic tools, including programs such as BLAST, ClustalX, Sequencher, and etc. Even though the two or more sequences may not match exactly and/or do not have the same length, an alignment of the sequences can still be performed and, if desirable, a "consensus" sequence generated. Indeed, programs and algorithms used for alignments typically tolerate definable levels of differences, including insertions, deletions, inversions, polymorphisms, point mutations, etc. Such alignments can aid in the determination of which positions in one nucleotide sequence correspond to which positions in other nucleotide sequences. The abbreviation "CpG" is used herein to refer to a dinucleotide comprised of a cytosine nucleotide (deoxycytidine) linked via a phosphate group to a guanine nucleotide (deoxyguanosine) through linkages to the 5' position of the deoxycytidine and the 3' position of the deoxyguanosine. The cytosine in this dinucleotide is said to be in the "5' position" of the dinucleotide, and the guanine is said to be in the "3' position" of the dinucleotide. As is understood by one of ordinary skill in the art, the abbreviation "CpG" also refers to modified dinucleotides similar, so long as the 5' nucleotide is still identifiable as deoxycytidine and the 3' nucleotide is still identifiable as deoxyguanosine. For example, a deoxycytidine-deoxyguanosine dinucleotide in which the cytosine ring is methylated at the 5 position is still considered a CpG dinucleotide, and may be referred to as a methylated CpG or abbreviated as 5mCpG. As used herein, the abbreviation CpG can also refer to a CpG site, defined below.

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 kb in length.

The term "Observed/Expected Ratio" ("O/E Ratio") refers to the frequency of CpG dinucleotides within a particular DNA sequence, and corresponds to the (number of CpG sites/(number of C bases X number of G bases)) X band length for each fragment.

The term "CpG site" is used herein to refer to a position within a region of DNA corresponding to a position where a CpG dinucleotide is found in a reference sequence. One of ordinary skill in the art will understand the term CpG site to encompass the location in the region of DNA where a CpG dinucleotide is typically found, whether or not the dinucleotide at that position is a CpG dinucleotide in a particular DNA molecule. For examples, the DNA sequence of a gene may typically contain a CpG dinucleotide at a particular position, but may contain other dinucleotides at the corresponding position in mutant versions, polymorphic variants, or other variations of the gene. Some mutations such as single base substitutions may alter the identity of the dinucleotide to be something other than CpG. Other mutations such as insertions or deletions may alter the position of the CpG dinucleotide typically found at a particular site. In cases such as these, the term CpG site is understood by one of ordinary skill in the art to encompass the site corresponding to the position where a CpG dinucleotide is typically found, for example, in individuals not carrying a mutation at this location. Similarly, the term CpG site also encompasses the corresponding site in a nucleic acid that has been modified experimentally, for example by labeling, methylation, demethylation, deamination (including conversion of a cytosine to uracil by a chemical such as sodium bisulfite), etc.

The term "predicting the sensitivity to a treatment", as used herein refers to the determination of the likelihood that the patient will respond either favorably or unfavorably to a given therapy. Especially, the term "prediction", as used herein, relates to an individual assessment of any parameter that can be useful in determining the evolution of a patient. Preferred confidence intervals are at least 50%, at least 60%, at least 70%, at least 80%, at least 90% at least 95%. The p- values are, preferably, 0.2, 0.1 or 0.05.

### Detailed description of certain embodiments of the invention

### Methylation assays

The methylation status of a DMR in the DNA of a biological sample may be determined using any methylation assay. Several quantitative methylation assays are commercially available. These include COBRA™ (Ziong and Laird, Nucleic Acid Res 1997 25; 2532-4) which uses methylation sensitive restriction endonuclease, gel electrophoresis and detection based on labeled hybridization probes. Another available technique is the Methylation Specific PCR (MSP) for amplification of DNA segments of interest. This is performed after sodium 'bisulfite' conversion of cytosine using methylation sensitive probes. MethyLight™, a quantitative methylation assay-based uses fluorescence based PCR (Eads et al, Cancer Res 1999; 59:2302-2306). Another method used is the Quantitative Methylation (QMTM) assay, which combines PCR amplification with fluorescent probes designed to bind to putative methylation sites. Ms-SNuPE™ is a quantitative technique for determining differences in methylation levels in CpG sites. As with other techniques bisulfite treatment is first performed leading to the conversion of unmethylated cytosine to uracil while methyl cytosine is unaffected. PCR primers specific for bisulfite converted DNA is used to amplify the target sequence of interest. The amplified PCR product is isolated and used to quantitate the methylation status of the CpG site of interest (Gonzalgo and Jones Nuclei Acids Res 1997; 25:252-31). The preferred method of measurement of cytosine methylation is the Illumina method.

### Illumina method

For DNA methylation assay the Illumina Infinium® Infinium MethylationEPIC Beadchip assay is preferably used for genome wide quantitative methylation profiling. Briefly genomic DNA is extracted from cells in this case whole blood, for which the original source of the DNA is preferably white blood cells. Using techniques widely known in the trade, the genomic DNA may be isolated using commercial kits. Proteins and other contaminants are preferably removed from the DNA, preferably using proteinase K. The DNA may be removed from the solution using available methods such as organic extraction, salting out or binding the DNA to a solid phase support.

### Bisulfite Conversion

As described in the Infinium® Assay Methylation Protocol Guide, prior to its analysis, DNA must be treated with sodium bisulfite which converts unmethylated cytosine to uracil, while the methylated cytosine remains unchanged. The bisulfite converted DNA is then denatured and neutralized. The genomic DNA may be bisulfite converted using commercial kits, e.g ZYMO®. The denatured DNA is then amplified. The whole genome application process increases the amount of DNA by up to several thousand-fold. The next step uses enzymatic means to fragment the DNA. The fragmented DNA is next precipitated using isopropanol and separated by centrifugation. The separated DNA is next suspended in a hybridization buffer. The fragmented DNA is then hybridized to beads that have been covalently limited to 50 mer nucleotide segments at a locus specific to the cytosine nucleotide of interest in the genome. There are a total of over 850,000 bead types specifically designed to anneal to the locus where the particular cytosine is located. The beads are bound to silicon based arrays. There are two bead types designed for each locus, one bead type represents a probe that is designed to match to the methylated locus at which the cytosine nucleotide will remain unchanged. The other bead type corresponds to an initially unmethylated cytosine which after bisulfite treatment is converted to a thiamine nucleotide. Unhybridized (not annealed to the beads) DNA is washed away leaving only DNA segments bound to the appropriate bead and containing the cytosine of interest. The bead bound oligomer, after annealing to the corresponding patient DNA sequence, then undergoes single base extension with fluorescently labeled nucleotide using the 'overhang' beyond the cytosine of interest in the patient DNA sequence as the template for extension.

If the cytosine of interest is unmethylated then it will match perfectly with the unmethylated or "U" bead probe. This enables single base extensions with fluorescent labeled nucleotide probes and generate fluorescent signals for that bead probe that can be read in an automated fashion. If the cytosine is methylated, single base mismatch will occur with the "U" bead probe oligomer. No further nucleotide extension on the bead oligomer occurs however thus preventing incorporation of the fluorescent tagged nucleotides on the bead. This will lead to low fluorescent signal form the bead "U" bead. The reverse will happen on the "M" or methylated bead probe.

Laser is used to stimulate the fluorophore bound to the single-base used for the sequence extension. The level of methylation at each cytosine locus is determined by the intensity of the fluorescence from the methylated compared to the unmethylated bead. Cytosine methylation level is expressed as "W' which is the ratio of the methylated-bead probe signal to total signal intensity at that cytosine locus. These techniques for determine cytosine methylation have been previously described and are widely available for commercial use.

In preferred embodiments of the invention, the detected methylation status is hypermethylation, an increase in the methylation of cytosines in CpG sites in responders compared to non-responders. The increase can be about 1%, about 5%, about 10%, about 20%, about 25%, about 30%, about 35%, about 40%, about 45%, about 50%, about 60%, about 65%, about 70%, about 75%, about 80%, about 85%, about 90%, about 95%, or more than about 95% greater than the extent of methylation of cytosines typically expected or observed for the CpG site or sites being evaluated. In preferred embodiments of the invention, the detected methylation status is hypomethylation, a decrease in the methylation of cytosines in CpG sites in responders compared to non-responders. The decrease can be about 1%, about 5%, about 10%, about 20%, about 25%, about 30%, about 35%, about 40%, about 45%, about 50%, about 60%, about 65%, about 70%, about 75%, about 80%, about 85%, about 90%, about 95%, or more than about 95% less than the extent of methylation of cytosines typically expected or observed for the CpG site or sites being evaluated. Suitable controls may need to be incorporated in order to ensure the method chosen is working correctly and reliably. Suitable controls may include assessing the methylation status of a gene known to be methylated. This experiment acts as a positive control to ensure that false negative results are not obtained. The DMR may be one which is known to be methylated in the sample under investigation or it may have been artificially methylated. In one embodiment, the DMR of interest may be assessed in normal cells, following treatment with SssI methyltransferase, as a positive control. Additionally or alternatively, suitable negative controls may be employed with the methods of the invention. Here, suitable controls may include assessing the methylation status of a gene known to be unmethylated or a DMR that has been artificially demethylated. This experiment acts as a negative control to ensure that false positive results are not obtained. In one embodiment, the DMR of interest may be assessed in normal cells as a negative control, in particular if the DMR is unmethylated in normal tissues.

In a preferred embodiment, the method according to the invention is determined, by determining if hyper- or hypomethylation is determined in at least two, preferably three, four or five DMRs as disclosed herein. An advantage thereof is that if more of said DMRs are hyper or hypomethylated, the sensitivity of the method is greater.

In certain embodiments of the invention, the detected methylation status is determined in part or wholly on the basis of a comparison with a control. The control can be a value or set of values related to the extent and/or pattern of methylation in a control sample. In certain embodiments of the invention, such a value or values may be determined, for example, by calculations, using algorithms, and/or from previously acquired and/or archived data. In certain embodiments of the invention, the value or set of values for the control is derived from experiments performed on samples or using a subject. For example, control data can be derived from experiments on samples derived from biological samples form subjects known to be sensitive to an anti-inflammatory treatment against IBD, using a monoclonal antibody targeting tumor necrosis factor α or integrin α4β7.

The term "control value" as used herein refers to a value representing the quantity of methylation of said DMR in a non-responder (e.g., from white blood cells from a non-responder), thereby quantifying the average methylation density in the locus compared to the methylation density of the control DNA. A skilled person will understand that it is also possible to compare the methylation of a certain DMR with the methylation level of the same DMR in a responder. In such case, when the methylation status is at a similar level compared to a control representative of a responder (which could be increased or decreased compared to a non-responder), this indicates that a subject is sensitive to a treatment. Using control values of methylation in DMRs of responders is also within the scope of this invention.

In certain embodiments of the invention, a control comprising DNA that is mostly or entirely demethylated, at one or more of the CpG sites being analyzed, is used. Such a control might be obtained, for example, from mutant tissues or cells lacking methyltransferase activity and/or from tissues or cells that have been chemically demethylated. For example, controls may be obtained from tissues or cells lacking activity of methyltransferases Dnmtl, Dnmt2, Dnmt3a, Dnmt3b, or combinations thereof. Agents such as 5-aza-2'-deoxycytidine may be used to chemically demethylate DNA.

In certain embodiments of the invention, a control comprising DNA that is mostly or entirely methylated, at one or more of the CpG sites being analyzed, is used. Such a control might be obtained, for example, from cells or tissues that are known or expected to be mostly or entirely methylated at the CpG site or sites of interest. Such a control could also be obtained by cells or tissues in which methylation levels have been altered and/or manipulated, for example, by overexpression of methyltransferases (such as enzymes Dnmtl, Dnmt2, Dnmt3a, Dnmt3b, any of the bacterial 5-CpG methyltransferases, or combinations thereof). In certain embodiments of the invention, samples used to obtain control values are processed and/or manipulated in the same manner as the samples being evaluated. In a preferred embodiment, said control comprises a colorectal cancer cell line with a known methylation status.

In some embodiments, negative control samples are generated by treating positive control samples with a demethylating agent, preferably 5-aza-2'-deoxycytidine.

### Medical treatment using infliximab, vedoluzimab or adalumimab

In another aspect, the invention provides Infliximab for use in the treatment of a subject who is sensitive to a treatment with infliximab in the treatment as determined using the method according to the invention. If a positive clinical response to treatment with infliximab is determined, the patient is identified or selected for a treatment with infliximab. Preferably, one or more response criteria as described herein above are used to determine whether there is positive clinical response. If a negative clinical response to a treatment is determined, the patient is not selected for treatment, and one or more alternative drug or medical intervention may be more beneficial for the treatment of IBD.

In another aspect, the invention provides vedoluzimab for use in the treatment of a subject who is sensitive to a treatment with vedoluzimab in the treatment as determined using the method according to the invention. If a positive clinical response to treatment with vedoluzimab is determined, the patient is identified or selected for a treatment with vedoluzimab. If a negative clinical response to a treatment is determined, the patient is not selected for treatment, and one or more alternative drug or medical intervention may be more beneficial for the treatment of IBD.

In another aspect, the invention provides Adalimumab for use in the treatment of a subject who is sensitive to a treatment with Adalimumab in the treatment as determined using the method according to the invention. If a positive clinical response to treatment with Adalimumab is determined, the patient is identified or selected for a treatment with Adalimumab. If a negative clinical response to a treatment is determined, the patient is not selected for treatment, and one or more alternative drug or medical intervention may be more beneficial for the treatment of IBD.

The above disclosure generally describes the present invention. Unless defined otherwise, all technical and scientific terms used herein have the same meaning as those commonly understood by one of ordinary skill in the art to which this invention belongs. A more complete understanding can be obtained by reference to the following specific examples which are provided herein for purposes of illustration only, and are not intended to limit the scope of the invention.

### EXAMPLE SECTION

### Cohort assembly and preliminary classification

To investigate the differences in methylation, we assembled three separate cohorts (vedolizumab, adalimumab and infliximab). Blood samples were obtained from CD patients prior to (time point 1) and eight weeks after (time point 2) the start of their treatment. Patients were labeled as either responder or non-responders based on mucosal healing as observed by endoscopy and histology.

### Infinium Methylation Assay

Genome-wide screening of DNA methylation patterns was performed by using the Infinium MethylationEPIC BeadChips (Illumina, San Diego, US), allowing the simultaneous quantitative measurement of the methylation status over 850,000 CpG sites. By combining Infinium I and Infinium II assay chemistry technologies, the BeadChip provides coverage of 99% of RefSeq genes and 96 % of CpG islands. The Infinium MethylationEPIC BeadChip builds upon the industry-leading Infinium HumanMethylation450 BeadChip with > 90% of the original CpGs plus an additional 350,000 CpGs in enhancer regions.

DNA concentrations were determined using PicoGreen (Life Technologies, Darmstadt, Germany). The quality of genomic DNA samples was checked by agarose-gel analysisDNA (500 ng genomic DNA -) from each sample was bisulfite converted using the EZ-96 DNA Methylation Kit (Zymo Research Corporation, Orange, US) according to the manufacturer recommendations. Bisulfite treatment leads to the deamination of non-methylated cytosines to uracils, while methylated cytosines are refractory to the effects of bisulfite and remain cytosine.

Each sample was whole genome amplified and enzymatically fragmented following the instructions in the Illumina Infinium HD Assay Methylation Protocol Guide (genomic DNA). The DNA was applied to Infinium MethylationEPIC BeadChip and hybridization is performed for 16-24h at 48°C. During hybridization, the DNA molecules anneal to locus-specific DNA oligomers linked to individual bead types. One or two probes are used to interrogate CpG locus, depending on the probe design for a particular CpG site.

Allele-specific primer annealing is followed by single-base extension using DNP- and Bio tin- labeled ddNTPs. For Infinium I assay design, both bead types (one each for the methylated and unmethylated states) for the same CpG locus incorporate the same type of labeled nucleotide, determined by the base preceding the interrogated "C" in the CpG locus and therefore are detected in the same color channel. Infinium II uses only one bead type with a unique type of probe allowing detection of both alleles. The methylated and unmethylated signals are generated respectively in the green and the red channels.

After extension, the array is fluorescently stained, scanned, and the intensities at each CpGs were measured. Microarray scanning was done using an iScan array scanner (Illumina). DNA methylation values, described as beta values, are recorded for each locus in each sample. DNA methylation beta values are continuous variables between 0 and 1 , representing the percentage of methylation of a given cytosine corresponding to the ratio of the methylated signal over the sum of the methylated and unmethylated signals.

### Methylation analysis

Analysis of the methylation data was performed in the R statistical programming environment (27) (v3.5.1) using the Bioconductor (v3.7) packages minfi (28) (v1.26.2) for import, MethylAid (29) (v1.14.0), shinyMethyl (28) (v1.16.0), and ewastools (30) (v1.4) for quality control, functional normalization for normalization (31), limma (32,33) (v3.34.9) for linear regressions to find differentially methylated probes (DMPs). Samples were included in this study if they passed the detection p-value quality control and were not an outlier according to the principal component analysis. Probes were excluded from the analyses if the probe was found to be promiscuous (36). Furthermore, as both males and females were present in the cohort, all probes annotated to allosomes were removed. In addition to using the Illumina annotation of the probes, probes were also annotated to genes according to the promoter-capture Hi-C data from Javierre et al. (37). Linear regressions were performed on the M-values, whereas the Beta-values were utilized for the visualization (38). Age and sex were included as covariates in the DMP analyses. Plots were made using ggplot2 (v2.2.1) (39).

### Results

### Exploratory data analysis does not display large differences according to response

Evaluation of the first two principal components revealed no observable separation between the responders and non-responders. Specifically, regressing sex, time point, and drug response displayed no strong correlation (r2 > 0.5) with any of the first five principal components, suggesting that the known metadata were not associated to global changes in DNA methylation. Age was significantly associated with the first principal component of the vedolizumab data (r = 0.48; p = 2.17E-4). We included age and sex as covariates in our subsequent linear models.

As our sample pertained peripheral blood, we estimated the blood cell distribution and investigated whether any association with time of measurement and response was observable. We did not detect any significant association with time of measurement or response with blood cell distribution. The estimated blood cell counts were therefore not included as covariates in our subsequent linear models.

### Differential methylation observed between responders and non-responders

We next performed comparative analyses where we investigated whether differentially methylated probes (DMPs) were observable across response, time and an interaction of response and time. Comparing the responders with the non-responders yielded 79, 221, and 235 DMPs that were different for vedolizumab, adalimumab and infliximab respectively. Evaluation of these DMPs, based on log-odds (B) and the delta (difference of mean beta-value between groups) yielded 9, 11, and 38 DMPs indicative for vedolizumab, adalimumab and infliximab response respectively (Table 1). None of the drugs displayed any significant changes in DNA methylation across time for responders or non-responders separately. Similarly, no changes in DNA methylation were observed when comparing responders with non-responders across time. Our results therefore indicate that the DNA methylome of responders and non-responders are different a priori and that such differences remain stable across the measured timespan.

**Table 1**

| **SEQ-ID** | **p-value** | **FDR** | **B** | **Methyl** | **µ_R_T1** | **SE_R_T1** | **µ_NR_T1** | **SE_NR_T1** | **Δ_T1** | **µ_R_T2** | **SE_R_T2** | **µ_NR_T2** | **SE_NR_T2** | **Δ_T2** |
|---|---|---|---|---|---|---|---|---|---|---|---|---|---|---|
| 1 | 2,86E-20 | 7,68E-15 | 17,56 | R > NR | 0,872 | 0,009 | 0,271 | 0,013 | 0,600 | 0,892 | 0,006 | 0,295 | 0,008 | 0,597 |
| 2 | 2,77E-11 | 3,90E-06 | 11,45 | R < NR | 0,046 | 0,005 | 0,186 | 0,026 | -0,140 | 0,047 | 0,004 | 0,161 | 0,025 | -0,114 |
| 3 | 3,15E-10 | 3,17E-05 | 10,13 | R > NR | 0,821 | 0,047 | 0,443 | 0,037 | 0,377 | 0,835 | 0,044 | 0,426 | 0,017 | 0,408 |
| 4 | 7,25E-10 | 6,49E-05 | 9,64 | R < NR | 0,078 | 0,006 | 0,236 | 0,031 | -0,158 | 0,083 | 0,010 | 0,246 | 0,031 | -0,163 |
| 5 | 1,25E-09 | 1,01E-04 | 9,31 | R > NR | 0,842 | 0,008 | 0,539 | 0,083 | 0,303 | 0,832 | 0,010 | 0,549 | 0,065 | 0,283 |
| 6 | 5,33E-09 | 3,58E-04 | 8,41 | R < NR | 0,057 | 0,015 | 0,259 | 0,027 | -0,201 | 0,062 | 0,011 | 0,282 | 0,032 | -0,219 |
| 7 | 7,79E-09 | 4,18E-04 | 8,16 | R > NR | 0,893 | 0,014 | 0,598 | 0,100 | 0,295 | 0,889 | 0,019 | 0,569 | 0,110 | 0,320 |
| 8 | 6,83E-09 | 4,18E-04 | 8,25 | R < NR | 0,350 | 0,016 | 0,501 | 0,024 | -0,151 | 0,362 | 0,013 | 0,486 | 0,018 | -0,124 |
| 9 | 7,28E-09 | 4,18E-04 | 8,21 | R < NR | 0,335 | 0,067 | 0,808 | 0,016 | -0,473 | 0,348 | 0,068 | 0,797 | 0,022 | -0,449 |
| 10 | 1,94E-08 | 9,21E-04 | 7,56 | R > NR | 0,754 | 0,024 | 0,566 | 0,033 | 0,188 | 0,754 | 0,021 | 0,579 | 0,052 | 0,175 |
| 11 | 3,32E-08 | 1,41E-03 | 7,20 | R < NR | 0,313 | 0,019 | 0,541 | 0,026 | -0,229 | 0,348 | 0,018 | 0,534 | 0,009 | -0,187 |
| 12 | 1,41E-07 | 3,65E-03 | 6,20 | R < NR | 0,213 | 0,012 | 0,308 | 0,005 | -0,095 | 0,238 | 0,009 | 0,302 | 0,009 | -0,064 |
| 13 | 3,00E-07 | 6,56E-03 | 5,66 | R < NR | 0,243 | 0,022 | 0,522 | 0,050 | -0,280 | 0,231 | 0,024 | 0,505 | 0,034 | -0,275 |
| 14 | 3,15E-07 | 6,67E-03 | 5,62 | R < NR | 0,526 | 0,015 | 0,688 | 0,027 | -0,162 | 0,523 | 0,020 | 0,698 | 0,009 | -0,174 |
| 15 | 4,59E-07 | 8,59E-03 | 5,35 | R > NR | 0,271 | 0,027 | 0,125 | 0,017 | 0,146 | 0,273 | 0,025 | 0,101 | 0,007 | 0,172 |
| 16 | 4,96E-07 | 8,76E-03 | 5,29 | R < NR | 0,230 | 0,021 | 0,434 | 0,024 | -0,204 | 0,250 | 0,016 | 0,423 | 0,013 | -0,173 |
| 17 | 5,06E-07 | 8,76E-03 | 5,28 | R < NR | 0,424 | 0,017 | 0,581 | 0,017 | -0,158 | 0,442 | 0,018 | 0,541 | 0,015 | -0,099 |
| 18 | 5,25E-07 | 8,80E-03 | 5,25 | R < NR | 0,241 | 0,017 | 0,441 | 0,033 | -0,199 | 0,250 | 0,015 | 0,407 | 0,044 | -0,157 |
| 19 | 7,09E-07 | 9,91E-03 | 5,03 | R > NR | 0,550 | 0,023 | 0,396 | 0,024 | 0,154 | 0,543 | 0,021 | 0,376 | 0,030 | 0,167 |
| 20 | 6,19E-07 | 9,91E-03 | 5,13 | R > NR | 0,607 | 0,017 | 0,482 | 0,013 | 0,125 | 0,625 | 0,015 | 0,471 | 0,018 | 0,154 |
| 21 | 6,39E-07 | 9,91E-03 | 5,11 | R < NR | 0,139 | 0,023 | 0,279 | 0,025 | -0,140 | 0,136 | 0,026 | 0,262 | 0,026 | -0,126 |
| 22 | 8,33E-07 | 1,11E-02 | 4,91 | R < NR | 0,258 | 0,021 | 0,431 | 0,039 | -0,174 | 0,258 | 0,022 | 0,415 | 0,029 | -0,157 |
| 23 | 8,39E-07 | 1,11E-02 | 4,90 | R > NR | 0,477 | 0,035 | 0,222 | 0,058 | 0,255 | 0,456 | 0,015 | 0,237 | 0,068 | 0,219 |
| 24 | 1,26E-06 | 1,34E-02 | 4,60 | R < NR | 0,654 | 0,023 | 0,782 | 0,016 | -0,128 | 0,660 | 0,025 | 0,793 | 0,021 | -0,133 |
| 25 | 1,34E-06 | 1,34E-02 | 4,56 | R > NR | 0,369 | 0,015 | 0,251 | 0,005 | 0,118 | 0,380 | 0,018 | 0,246 | 0,023 | 0,134 |
| 26 | 1,87E-06 | 1,64E-02 | 4,30 | R > NR | 0,599 | 0,046 | 0,372 | 0,013 | 0,227 | 0,628 | 0,044 | 0,337 | 0,024 | 0,291 |
| 27 | 3,09E-06 | 2,28E-02 | 3,92 | R < NR | 0,200 | 0,017 | 0,346 | 0,021 | -0,147 | 0,225 | 0,014 | 0,354 | 0,011 | -0,129 |
| 28 | 4,42E-06 | 2,76E-02 | 3,65 | R < NR | 0,708 | 0,015 | 0,836 | 0,008 | -0,128 | 0,725 | 0,023 | 0,833 | 0,010 | -0,108 |
| 29 | 5,03E-06 | 3,00E-02 | 3,55 | R < NR | 0,550 | 0,017 | 0,691 | 0,022 | -0,141 | 0,569 | 0,016 | 0,676 | 0,032 | -0,106 |
| 30 | 6,40E-06 | 3,50E-02 | 3,36 | R > NR | 0,596 | 0,012 | 0,496 | 0,042 | 0,099 | 0,600 | 0,010 | 0,475 | 0,044 | 0,126 |
| 31 | 6,28E-06 | 3,50E-02 | 3,38 | R < NR | 0,110 | 0,011 | 0,314 | 0,029 | -0,204 | 0,157 | 0,026 | 0,291 | 0,031 | -0,133 |
| 32 | 6,82E-06 | 3,57E-02 | 3,31 | R > NR | 0,910 | 0,006 | 0,843 | 0,016 | 0,067 | 0,907 | 0,010 | 0,841 | 0,009 | 0,065 |
| 33 | 9,73E-06 | 4,18E-02 | 3,04 | R > NR | 0,439 | 0,062 | 0,122 | 0,007 | 0,317 | 0,478 | 0,059 | 0,144 | 0,013 | 0,334 |
| 34 | 1,04E-05 | 4,23E-02 | 2,99 | R < NR | 0,578 | 0,014 | 0,675 | 0,024 | -0,097 | 0,552 | 0,022 | 0,685 | 0,030 | -0,133 |
| 35 | 1,22E-05 | 4,55E-02 | 2,86 | R < NR | 0,183 | 0,011 | 0,405 | 0,079 | -0,222 | 0,188 | 0,013 | 0,382 | 0,065 | -0,194 |
| 36 | 1,24E-05 | 4,55E-02 | 2,85 | R > NR | 0,807 | 0,010 | 0,657 | 0,034 | 0,150 | 0,786 | 0,019 | 0,669 | 0,024 | 0,118 |
| 37 | 1,27E-05 | 4,60E-02 | 2,83 | R > NR | 0,421 | 0,033 | 0,266 | 0,042 | 0,154 | 0,451 | 0,023 | 0,268 | 0,021 | 0,183 |
| 38 | 1,34E-05 | 4,66E-02 | 2,79 | R > NR | 0,378 | 0,020 | 0,246 | 0,025 | 0,132 | 0,358 | 0,014 | 0,255 | 0,020 | 0,102 |
| 39 | 1,14E-08 | 3,07E-03 | 7,13 | R < NR | 0,131 | 0,006 | 0,303 | 0,034 | -0,172 | 0,144 | 0,016 | 0,328 | 0,054 | -0,184 |
| 40 | 1,04E-07 | 7,74E-03 | 5,78 | R < NR | 0,212 | 0,021 | 0,403 | 0,019 | -0,191 | 0,199 | 0,027 | 0,402 | 0,023 | -0,203 |
| 41 | 1,53E-07 | 8,78E-03 | 5,54 | R > NR | 0,821 | 0,017 | 0,691 | 0,031 | 0,130 | 0,832 | 0,014 | 0,645 | 0,017 | 0,187 |
| 42 | 3,39E-07 | 1,21E-02 | 5,02 | R > NR | 0,873 | 0,016 | 0,696 | 0,021 | 0,177 | 0,872 | 0,017 | 0,706 | 0,027 | 0,165 |
| 43 | 3,64E-07 | 1,21E-02 | 4,97 | R > NR | 0,845 | 0,023 | 0,615 | 0,020 | 0,230 | 0,851 | 0,022 | 0,643 | 0,026 | 0,208 |
| 44 | 3,72E-07 | 1,21E-02 | 4,96 | R > NR | 0,913 | 0,007 | 0,526 | 0,097 | 0,387 | 0,917 | 0,007 | 0,512 | 0,104 | 0,406 |
| 45 | 7,73E-07 | 1,89E-02 | 4,47 | R < NR | 0,614 | 0,063 | 0,966 | 0,003 | -0,352 | 0,625 | 0,061 | 0,967 | 0,003 | -0,342 |
| 46 | 4,20E-06 | 4,70E-02 | 3,30 | R > NR | 0,354 | 0,017 | 0,255 | 0,025 | 0,098 | 0,404 | 0,038 | 0,230 | 0,022 | 0,173 |
| 47 | 4,68E-06 | 4,87E-02 | 3,22 | R > NR | 0,827 | 0,022 | 0,712 | 0,031 | 0,115 | 0,809 | 0,011 | 0,707 | 0,021 | 0,103 |
| 48 | 4,09E-15 | 1,37E-09 | 12,79 | R > NR | 0,269 | 0,011 | 0,044 | 0,003 | 0,225 | 0,284 | 0,015 | 0,049 | 0,005 | 0,235 |
| 49 | 2,27E-06 | 2,04E-02 | 3,98 | R > NR | 0,820 | 0,015 | 0,647 | 0,024 | 0,173 | 0,817 | 0,022 | 0,646 | 0,028 | 0,171 |
| 50 | 3,70E-06 | 2,72E-02 | 3,63 | R < NR | 0,424 | 0,044 | 0,591 | 0,028 | -0,167 | 0,495 | 0,045 | 0,633 | 0,027 | -0,138 |
| 51 | 4,66E-06 | 3,02E-02 | 3,46 | R < NR | 0,157 | 0,013 | 0,347 | 0,036 | -0,190 | 0,181 | 0,024 | 0,369 | 0,030 | -0,188 |
| 52 | 5,37E-06 | 3,25E-02 | 3,36 | R > NR | 0,467 | 0,011 | 0,401 | 0,011 | 0,066 | 0,458 | 0,008 | 0,385 | 0,012 | 0,073 |
| 53 | 5,51E-06 | 3,30E-02 | 3,34 | R > NR | 0,858 | 0,009 | 0,789 | 0,015 | 0,068 | 0,866 | 0,008 | 0,781 | 0,009 | 0,085 |
| 54 | 7,16E-06 | 3,55E-02 | 3,15 | R < NR | 0,341 | 0,016 | 0,451 | 0,004 | -0,109 | 0,354 | 0,024 | 0,457 | 0,014 | -0,103 |
| 55 | 9,61E-06 | 4,17E-02 | 2,93 | R > NR | 0,409 | 0,032 | 0,243 | 0,022 | 0,166 | 0,434 | 0,027 | 0,253 | 0,026 | 0,182 |
| 56 | 1,08E-05 | 4,48E-02 | 2,85 | R > NR | 0,579 | 0,025 | 0,448 | 0,013 | 0,131 | 0,583 | 0,021 | 0,463 | 0,012 | 0,120 |
| 57 | 1,14E-05 | 4,54E-02 | 2,81 | R < NR | 0,271 | 0,027 | 0,521 | 0,047 | -0,249 | 0,280 | 0,033 | 0,523 | 0,038 | -0,243 |
| 58 | 1,30E-05 | 4,81E-02 | 2,71 | R < NR | 0,497 | 0,026 | 0,623 | 0,010 | -0,125 | 0,487 | 0,030 | 0,602 | 0,011 | -0,115 |

### Abbreviations used in Table 1

- p: P-value
- ab: Antibody
- (FDR: Multiple tests adjusted P-value (false discovery rate)
- B: Log-odds of the differentially methylated position
- Meth: Direction of effect relative to responders; higher or less methylated
- *µ_*R_T1: Mean methylation index (beta-value) in responders at baseline (timepoint 1)
- SE_R_T1: Standard error of methylation index (beta-value) in responders at baseline (timepoint 1)
- *µ*_NR_T1: Mean methylation index (beta-value) in non-responders at baseline (timepoint 1)
- SE_NR_T1: Standard error of methylation index (beta-value) in non-responders at baseline (timepoint 1)
- Δ_T1: Difference of the mean methylation index (beta-value) between responders and non-responders at baseline (timepoint 1)
- *µ*_R_T2: Mean methylation index (beta-value) in responders at follow up (timepoint 2)
- SE_R_T2: Standard error of methylation index (beta-value) in responders at follow up (timepoint 2)
- *µ*_NR_T2: Mean methylation index (beta-value) in non-responders at follow up (timepoint 2)
- SE_NR_T2: Standard error of methylation index (beta-value) in non-responders at follow up (timepoint 2)
- Δ_T2: Difference of the mean methylation index (beta-value) between responders and non-responders at follow up (timepoint 2)

### Sampling of cohorts

All CD patients scheduled for colonoscopy to assess disease activity because of clinical symptoms were asked to participate in this project and were recruited between 2009 and 2012. Patients could be included when active disease was confirmed during colonoscopy and/or MREnterography, and received remission induction therapy with prednisone or anti-TNF antibodies (infliximab or adalimumab). Before colonoscopy, peripheral blood was drawn for DNA and plasma isolation and for the measurement of CRP, leucocytes and albumin. In addition, the CD Activity Index (CDAI) and (Inflammatory Bowel Disease Questionnaire) IBDQ were obtained from every patient. During colonoscopy the CDEIS was scored and biopsies were collected from inflamed and non-inflamed regions of the intestine. At 8 weeks (ADA) or 14 weeks (VEDO and INFLX) patients again completed the CDAI and IBDQ and a second colonoscopy and blood drawing (DNA isolation and before mentioned plasma parameters measurements) was performed including scoring of the CDEIS and collection of biopsies.

We collected samples using strict inclusion and exclusion criteria. It should however been noted that that the sampling of the ADA cohort differed with the VEDO, INFLIXI protocol, especially with regard to the timespan between baseline and response qualification (8wks for ADA vs. 14wks for the other biologicals). A description of the sampling procedure for each of the three therapeutics was as follows:
Adalimumab: In total ten CD patients were assessed on their response towards adalimumab eight weeks after their treatment. CD patients were either labeled as responders (N=5, 3 female) or non-responders (N=5, 3 female), table 2. Blood samples were obtained through phlebotomy prior to and eight weeks after adalimumab treatment. Response to adalimumab was retrospectively accessed by means of serological/histologically assays and by means and colon endoscopy, prior and after treatment.

fold changes above 1 reflect improvement of IBDQ and fold changes below 1 reflect declined quality of life.

Vedolizumab: In total 11 CD patients and 1 UC patient were assessed on their response towards vedolizumab 14 weeks after their treatment. Patients were either labeled as responders (N=7, 3 female) or non-responders (N=5, all female), table 2. Blood samples were obtained through phlebotomy prior to and 14 weeks after vedolizumab treatment. Response to vedolizumab was retrospectively accessed by means of serological/histologically assays and by means and colon endoscopy, prior and after treatment.

Infliximab: In total 12 CD patients patient were assessed on their response towards Infliximab 14 weeks after their treatment. Patients were either labeled as responders (N=8, 4 female) or non-responders (N=4, two female), table 2. Blood samples were obtained through phlebotomy prior to and 14 weeks after infliximab treatment. Response to infliximab was retrospectively accessed by means of serological/histologically assays and by means and colon endoscopy, prior and after treatment.

**Table 2: Descriptives response cohorts ADA, VEDO, INFLIXI and USTEKI**

| | **ADA (8wk)** | **VEDO (14 wk)** | **INFLIXI (14 wk)** |
|---|---|---|---|
| **N** | 10 | 12 | 12 |
| **Diagnosis** | 10 CD | 11 CD / 1UC | 12 CD |
| | | | |
| **Nᵣₑₛₚ (female)** | 5(3) | 7(3) | 8(4) |
| **Nₙᵣₑₛₚ(female)** | 5(3) | 5(5) | 4(2) |
| | | | |
| **Nᵣₑₛₚ Mean age (SD)** | 38.6(14.9) | 23.8(13.5) | 35.9(11.6) |
| **Nₙᵣₑₛₚ Mean age (SD)** | 30.8(14.8) | 19.5(12.7) | 27(7.6) |

| | | | |
|---|---|---|---|
| For all samples in these three cohorts (ADA, VEDO and INFLIXI), genome-wide DNA-methylation profiles were obtained at both baseline (T=0 wks, start of ADA administration) and at follow-up (T=8 wks or 14 wks). | | | |

### References

1. Van Deventer SJ. Tumour necrosis factor and Crohn's disease. Gut (Internet). 1997;40:443-8. Available from: http://www.ncbi.nlm.nih.gov/pubmed/9176068
2. Meenan J, Spaans J, Grool TA, Pals ST, Tytgat GNJ, Van Deventer SJH. Altered expression of α4β7, a gut homing integrin, by circulating and mucosal T cells in colonic mucosal inflammation. Gut. 1997;
3. Gorfu G, Rivera-Nieves J, Ley K. Role of 7 Integrins in Intestinal Lymphocyte Homing and Retention. Curr. Mol. Med. 2009.
4. Yang Y, Cardarelli PM, Lehnert K, Rowland S, Krissansen GW. LPAM-1 (integrin α4β7)-ligand binding: Overlapping binding sites recognizing VCAM-1, MAdCAM-1 and CS-1 are blocked by fibrinogen, a fibronectin-like polymer and RGD-like cyclic peptides. Eur J Immunol. 1998;
5. Berlin C, Bargatze RF, Campbell JJ, Von Andrian UH, Szabo MC, Hasslen ∼S R, et al. a4 Integrins Mediate Lymphocyte Attachment and Rolling under Physiologic Flow. Cell. 1995.
6. Hynes RO. Integrins: bidirectional, allosteric signaling machines. Cell. United States; 2002;110:673-87.
7. Denmark VK, Mayer L. Current status of monoclonal antibody therapy for the treatment of inflammatory bowel disease: An update. Expert Rev. Clin. Immunol. 2013.
8. Gagniere C, Beaugerie L, Pariente B, Seksik P, Amiot A, Abitbol V, et al. Benefit of infliximab reintroduction after successive failure of infliximab and adalimumab in Crohn's disease. J Crohn's Colitis. 2015;
9. Chaparro M, Andreu M, Acosta MB de, García-Planella E, Ricart E, Domènech E, et al. Effectiveness of infliximab after adalimumab failure in Crohn's disease. World J Gastroenterol. 2012;
10. Antonio JR, Sanmiguel J, Cagnon GV, Augusto MSF, de Godoy MF, Pozetti EMO. Infliximab in patients with psoriasis and other inflammatory diseases: Evaluation of adverse events in the treatment of 168 patients. An Bras Dermatol. 2016;
11. Perdriger A. Infliximab in the treatment of rheumatoid arthritis. Biol. Targets Ther. 2009.
12. Grainger R, Harrison AA. Infliximab in the treatment of ankylosing spondylitis. Biol. Targets Ther. 2007.
13. Burmester GR, Panaccione R, Gordon KB, Mcllraith MJ, Lacerda APM. Adalimumab: Long-term safety in 23 458 patients from global clinical trials in rheumatoid arthritis, juvenile idiopathic arthritis, ankylosing spondylitis, psoriatic arthritis, psoriasis and Crohn's disease. Ann Rheum Dis. 2013;
14. Burmester GR, Landewé R, Genovese MC, Friedman AW, Pfeifer ND, Varothai NA, et al. Adalimumab long-term safety: Infections, vaccination response and pregnancy outcomes in patients with rheumatoid arthritis. Ann Rheum Dis. 2017;
15. Balzola F, Cullen G, Ho GT, Russell R. Vedolizumab as induction and maintenance therapy for Crohn's disease: Commentary. Inflamm. Bowel Dis. Monit. 2014.
16. Feagan BG, Schwartz D, Danese S, Rubin DT, Lissoos TW, Xu J, et al. Efficacy of vedolizumab in fistulising Crohn's disease: Exploratory analyses of data from GEMINI 2. J Crohn's Colitis. 2018;
17. Ha C, Kornbluth A. Vedolizumab as a treatment for Crohn's disease and ulcerative colitis. Gastroenterol Hepatol. 2014;
18. Cassinotti A, Ardizzone S, Porro GB. Adalimumab for the treatment of Crohn's disease. Biol. Targets Ther. 2008.
19. Lichtenstein GR, Panaccione R, Mallarkey G. Review: Efficacy and safety of adalimumab in Crohn's disease. Therap. Adv. Gastroenterol. 2008.
20. Chaparro M, Panes J, Garcia V, Merino O, Nos P, Domenech E, et al. Long-term durability of response to adalimumab in Crohn's disease. Inflamm Bowel Dis. England; 2012;18:685-90.
21. Kestens C, van Oijen MGH, Mulder CLJ, van Bodegraven AA, Dijkstra G, de Jong D, et al. Adalimumab and infliximab are equally effective for Crohn's disease in patients not previously treated with anti-tumor necrosis factor-α agents. Clin Gastroenterol Hepatol. 2013;
22. Dalal SR, Cohen RD. What to do when biologic agents are not working in inflammatory bowel disease patients. Gastroenterol Hepatol. 2015;
23. Sierra Morales M, Birdi S, Samaan MA, Brown-Clarke C, Duncan J, Stanton A, et al. P673 Vedolizumab dose escalation as a way of recapturing response in patients with inflammatory bowel disease. J Crohn's Colitis (Internet). 2018;12:S451-2. Available from: http://dx.doi.org/10.1093/ecco-jcc/jjx180.800
24. Baert F, Glorieus E, Reenaers C, D'Haens G, Peeters H, Franchimont D, et al. Adalimumab dose escalation and dose de-escalation success rate and predictors in a large national cohort of Crohn's patients. J Crohn's Colitis. 2013;
25. Coskun M, Olsen AK, Holm TL, Kvist PH, Nielsen OH, Riis LB, et al. TNF-α-induced down-regulation of CDX2 suppresses MEP1A expression in colitis. Biochim Biophys Acta - Mol Basis Dis (Internet). Elsevier B.V.; 2012;1822:843-51. Available from: http://dx.doi.org/10.1016/j.bbadis.2012.01.012
26. Billmeier U, Dieterich W, Neurath MF, Atreya R. Molecular mechanism of action of anti-tumor necrosis factor antibodies in inflammatory bowel diseases. World J. Gastroenterol. 2016.
27. Ihaka R, Gentleman R. R: A Language for Data Analysis and Graphics. J Comput Graph Stat (Internet). (American Statistical Association, Taylor & Francis, Ltd., Institute of Mathematical Statistics, Interface Foundation of America); 1996;5:299-314. Available from: http://www.jstor.org/stable/1390807
28. Aryee MJ, Jaffe AE, Corrada-Bravo H, Ladd-Acosta C, Feinberg AP, Hansen KD, et al. Minfi: A flexible and comprehensive Bioconductor package for the analysis of Infinium DNA methylation microarrays. Bioinformatics. 2014;30:1363-9.
29. van Iterson M, Tobi EW, Slieker RC, den Hollander W, Luijk R, Slagboom PE, et al. MethylAid: visual and interactive quality control of large Illumina 450k datasets. Bioinformatics (Internet). 2014;30:3435-7. Available from: http://www.ncbi.nlm.nih.gov/pubmed/25147358
30. Heiss JA, Just AC. Identifying mislabeled and contaminated DNA methylation microarray data: an extended quality control toolset with examples from GEO. Clin Epigenetics (Internet). Clinical Epigenetics; 2018;10:73. Available from: http://www.ncbi.nim.nih.gov/pubmed/29881472%0Ahttp://www.pubmedcentral.nih.gov/articierender.fcgi ?artid=PMC5984806
31. Fortin J-PP, Labbe A, Lemire M, Zanke BW, Hudson TJ, Fertig EJ, et al. Functional normalization of 450k methylation array data improves replication in large cancer studies. Genome Biol (Internet). England; 2014;15:503. Available from: http://genomebiology.biomedcentral.corn/articles/10.1186/sl3059-014-0503-2
32. Ritchie ME, Phipson B, Wu D, Hu Y, Law CW, Shi W, et al. limma powers differential expression analyses for RNA-sequencing and microarray studies. Nucleic Acids Res (Internet). 2015;43:e47-e47. Available from: https://academic.oup.com/nar/article-lookup/ doi/10.1093/nar/gkv007
33. Ritchie ME, Diyagama D, Neilson J, van Laar R, Dobrovic A, Holloway A, et al. Empirical array quality weights in the analysis of microarray data. BMC Bioinformatics (Internet). 2006;7:261. Available from: http://bmcbioinformatics.biomedcentral.com/articles/10.1186/1471-2105-7-261
34. Peters TJ, Buckley MJ, Statham AL, Pidsley R, Samaras K, V Lord R, et al. De novo identification of differentially methylated regions in the human genome. Epigenetics Chromatin (Internet). 2015;8:6. Available from: http://www.epigeneticsandchromatin.com/content/8/1/6
35. Yu G, Wang LG, He QY. ChIP seeker: An R/Bioconductor package for ChIP peak annotation, comparison and visualization. Bioinformatics. 2015;31:2382-3.
36. Zhou W, Laird PW, Shen H. Comprehensive characterization, annotation and innovative use of Infinium DNA methylation BeadChip probes. Nucleic Acids Res (Internet). 2016;gkw967. Available from: http://fdslive.oup.com/www.oup.com/pdf/production_in_progress.pdf
37. Javierre BM, Burren OS, Wilder SP, Kreuzhuber R, Hill SM, Sewitz S, et al. Lineage-Specific Genome Architecture Links Enhancers and Non-coding Disease Variants to Target Gene Promoters. Cell (Internet). United States; 2016;167:1369-1384.el9. Available from: http://linkinghub.elsevier.com/retrieve/pii/S0092867416313228
38. Du P, Zhang X, Huang C-C, Jafari N, Kibbe WA, Hou L, et al. Comparison of Beta-value and M-value methods for quantifying methylation levels by microarray analysis. BMC Bioinformatics (Internet). BioMed Central Ltd; 2010;11:587. Available from: http://www.biomedcentral.com/1471-2105/11/587
39. Wickham H. ggplot2: Elegant Graphics for Data Analysis (Internet). New York, NY: Springer-Verlag New York; 2009. Available from: http://ggplot2.org
40. Phipson B, Maksimovic J, Oshlack A. missMethyl: an R package for analyzing data from Illumina's HumanMethylation450 platform. Bioinformatics (Internet). 2015;btv560. Available from: https://academic.oup.com/bioinformatics/article-lookup/doi/10.1093/bi oinformatics/btv560
41. Ahsan M, Ek WE, Rask-Andersen M, Karlsson T, Lind-Thomsen A, Enroth S, et al. The relative contribution of DNA methylation and genetic variants on protein biomarkers for human diseases. PLoS Genet. 2017;
42. Daca-Roszak P, Pfeifer A, Zebracka-Gala J, Rusinek D, Szybińska A, Jarzab B, et al. Impact of SNPs on methylation readouts by Illumina Infinium HumanMethylation450 BeadChip Array: implications for comparative population studies. BMC Genomics (Internet). 2015;16:1003. Available from: http://www.ncbi.nlm.nih.gov/pubmed/26607064
43. Nuij VJAA, Peppelenbosch MP, Woude CJ, Fuhler GM. Genetic polymorphism in ATG16L1 gene is associated with adalimumab use in inflammatory bowel disease. J Transl Med. 2017;
44. Koder S, Repnik K, Ferkolj I, Pernat C, Skok P, Weersma RK, et al. Genetic polymorphism in ATG16L1 gene influences the response to adalimumab in Crohn's disease patients. Pharmacogenomics. 2015;
45. Jürgens M, Laubender RP, Hartl F, Weidinger M, Seiderer J, Wagner J, et al. Disease activity, ANCA, and IL23R genotype status determine early response to infliximab in patients with ulcerative colitis. Am J Gastroenterol. 2010;
46. Dahlén R, Magnusson MK, Bajor A, Lasson A, Ung KA, Strid H, et al. Global mucosal and serum cytokine profile in patients with ulcerative colitis undergoing anti-TNF therapy. Scand J Gastroenterol. 2015;50:1118-26.
47. Medrano LM, Taxonera C, González-Artacho C, Pascual V, Gómez-García M, Barreiro-De Acosta M, et al. Response to infliximab in Crohn's disease: Genetic analysis supporting expression profile. Mediators Inflamm. 2015;
48. Sandborn WJ, Rutgeerts P, Enns R, Hanauer SB, Jean-Frédé ;, Colombel R, et al. Adalimumab Induction Therapy for Crohn Disease Previously Treated with Infliximab A Randomized Trial (Internet). 2007. Available from: www.annals.org
49. Muzes G, Tulassay Z, Sipos F, Müzes G, Tulassay Z, Sipos F. Interplay of autophagy and innate immunity in Crohn's disease: A key immunobiologic feature. World J Gastroenterol. China; 2013;19:4447-54.
50. Levin AD, Koelink PJ, Bloemendaal FM, Vos ACW, D'Haens GR, Van Den Brink GR, et al. Autophagy contributes to the induction of anti-TNF induced macrophages. J Crohn's Colitis. 2016;
51. Severine Vermeire et al, Correlation Between the Crohn's Disease Activity and Harvey-Bradshaw Indices in Assessing Crohn's Disease Severity, Clinical Gastroenterology and Hepatology 2010;8:357-363

## Claims

1. A method of determining or predicting the sensitivity of a subject to an anti-inflammatory treatment against IBD, using an antibody inhibiting binding of tumor necrosis factor α to its receptor or an antibody blocking integrin α4β7, wherein said method is based on determining the methylation status in at least one differentially methylated region (DMR) in the DNA of a biological sample of a subject.

2. The method according to claim 1, comprising determining, in at least one DMR of said DNA, the methylation status at one or more CpGs located within one or more of the nucleotide sequences comprised in one or more of the respective DMRs of the present invention independently selected from the group consisting of:
a) cg11261447 (SEQ ID NO:1), cg07460376 (SEQ ID NO:2), cg05155812 (SEQ ID NO:3), cg08332658 (SEQ ID NO:4), cg26175789 (SEQ ID NO:5), cg01615818 (SEQ ID NO:6), cg09255157 (SEQ ID NO:7), cg14234990 (SEQ ID NO:8), cg04850148 (SEQ ID NO:9), cg00356897 (SEQ ID NO: 10), cg01847718 (SEQ ID NO:11), cg01257975 (SEQ ID NO:12), cg09163005 (SEQ ID NO:13), cg12806797 (SEQ ID NO:14), cg05014687 (SEQ ID NO:15), cg07112131 (SEQ ID NO:16), cg16322792 (SEQ ID NO:17), cg09169455 (SEQ ID NO:18), cg24491749 (SEQ ID NO:19), cg03856973 (SEQ ID NO:20), cg20649823 (SEQ ID NO:21), cg23831005 (SEQ ID NO:22), cg07068406 (SEQ ID NO:23), cg05871254 (SEQ ID NO:24), cg17758362 (SEQ ID NO:25), cg06524124 (SEQ ID NO:26), cg26127836 (SEQ ID NO:27), cg01249606 (SEQ ID NO:28), cg12754571 (SEQ ID NO:29), cg19736195 (SEQ ID NO:30), cg23133460 (SEQ ID NO:31), cg23043514 (SEQ ID NO:32), cg06277607 (SEQ ID NO:33), cg12418129 (SEQ ID NO:34), cg10564845 (SEQ ID NO:35), cg13959831 (SEQ ID NO:36), cg21593676 (SEQ ID NO:37), cg02847897 (SEQ ID NO:38),
b) cg13679303 (SEQ ID NO:39), cg14598338 (SEQ ID NO:40), cg06031268 (SEQ ID NO:41), cg20603222 (SEQ ID NO:42), cg26224354 (SEQ ID NO:43), cg06378142 (SEQ ID NO:44), cg06406458 (SEQ ID NO:45), cg05622025 (SEQ ID NO:46), cg09715000 (SEQ ID NO:47), or,
c) cg26913836 (SEQ ID NO:48), cg15488109 (SEQ ID NO:49), cg25873494 (SEQ ID NO:50), cg25123302 (SEQ ID NO:51), cg01379846 (SEQ ID NO:52), cg08944241 (SEQ ID NO:53), cg04377463 (SEQ ID NO:54), cg03299990 (SEQ ID NO:55), cg00683245 (SEQ ID NO:56), cg12793466 (SEQ ID NO:57), cg05894480 (SEQ ID NO:58)
or a nucleotide sequence present within about 2,000bp (such as within about 200bp) 5' or 3' thereof, or an allelic variant and/or complementary sequence of any of said nucleotide sequences.

3. The method according to claim 1 or 2, wherein a higher level of methylation of cg11261447, cg05155812, cg26175789, cg09255157, cg00356897, cg05014687, cg24491749, cg03856973, cg07068406, cg17758362, cg06524124, cg19736195, cg23043514, cg06277607, cg13959831, cg21593676c and cg02847897 in comparison to a control value is indicative of an increased sensitivity to a therapy using infliximab.

4. The method according to claim lor 2, wherein a lower level of methylation of cg07460376, cg08332658,cg01615818,cg14234990,cg04850148,cg01847718,cg01257975, cg09163005,cg12806797,cg07112131,cg16322792,cg09169455,cg20649823, cg23831005,cg05871254,cg26127836,cg01249606,cg12754571,cg23133460, cg12418129 and cg10564845 in comparison to a control value is indicative of an increased sensitivity to a therapy using infliximab.

5. The method according to claim 1 or 2, wherein a higher level of methylation of cg06031268, cg20603222, cg26224354, cg06378142, cg05622025, and cg09715000 in comparison to a control value is indicative of an increased sensitivity to a therapy using vedolizumab.

6. The method according to claim 1 or 2, wherein a lower level of methylation of cg13679303, cg14598338 and cg06406458 in comparison to a control value is indicative of an increased sensitivity to a therapy using vedolizumab.

7. The method according to claim 2, wherein a higher level of methylation of cg26913836, cg15488109, cg01379846, cg08944241, cg03299990 and cg00683245 in comparison to a control value is indicative of an increased sensitivity to a therapy using adalimumab.

8. The method according to claim 2, wherein a lower level of methylation of cg25873494, cg25123302, cg04377463, cg12793466 and cg05894480 in comparison to a control value is indicative of an increased sensitivity to a therapy using adalimumab.

9. Method according to any of claims 1 - 8, wherein said IBD is Crohn's disease.

10. Method according to any of claims 1 - 9, wherein said biological sample comprises white blood cells.

11. Method according to any of claims 1 - 10, wherein said methylation level is determined using DNA methylation array, preferably an Illumina BeadChip EPIC array.

12. Infliximab for use in the treatment of a subject who is sensitive to a treatment with infliximab as determined using the method according to any of claims 1- 4, 9-11.

13. Vedolizumab for use in the treatment of a subject who is sensitive to a treatment with vedolizumab as determined using the method according to any of claims 1-2, 5-6, and 9-11.

14. Adalimumab for use in the treatment of a subject who is sensitive to a treatment with Adalimumab as determined using the method according to any of claims 1-2, 7-11.

15. Use of a DNA methylation array for the performing the method according to any of claims 1-11.


SEQUENCE LISTING

<110> Academisch Medisch Centrum

<120> METHYLATION MARKERS FOR PREDICTING SENSITIVITY TO TREATMENT WITH
MONOCLONAL ANTIBODIES

<130> 2017-023-EP-PR

<160> 58

<170> BiSSAP 1.3.6

<210> 1
<211> 122
<212> DNA
<213> Artificial Sequence

<220>
<223> cg11261447

<400> 1
acctcatgat ccacccgcct cggcctccca aagtgctggg attacaggcg tgagccaccg 60

cgcccggccc catgtggttt ttatacttgc catgaaactg ttctttggga tattattttg 120

tt 122

<210> 2
<211> 122
<212> DNA
<213> Artificial Sequence

<220>
<223> cg07460376

<400> 2
ccctctctcg ggatgtcgga cccgacattc tcaccatttc taggtttccg ggggacctgg 60

cgtcttagct atggatcgcc aatacctgca ggtcagaggg ccatagaggc tgggcctcta 120

ga 122

<210> 3
<211> 122
<212> DNA
<213> Artificial Sequence

<220>
<223> cg05155812

<400> 3
ggagactgag gcaggagaat cgcttgaacc cggcaggcag aagttgcaat gagccgagat 60

cgcgcaaatg cactccagcc caggcgatgg tgagagactc cgtttcaaaa aaaaaaaaaa 120

aa 122

<210> 4
<211> 122
<212> DNA
<213> Artificial Sequence

<220>
<223> cg08332658

<400> 4
ggacacagtc actgagcagg gaggagaaag gacgcccggg gggctggctg tcagcgcagc 60

cgccatctta tggtccaagt ggactgaggc cgcctgggcg aggagaactc agggcgcaaa 120

tt 122

<210> 5
<211> 122
<212> DNA
<213> Artificial Sequence

<220>
<223> cg26175789

<400> 5
tcaatgacaa gtcacagctg catagacgca gccccatcta tgtgctgact caggaggtaa 60

cgcagtgctt cctctctttg attaccaggg gtcttgactt ctgtactaaa gtaagagaga 120

aa 122

<210> 6
<211> 122
<212> DNA
<213> Artificial Sequence

<220>
<223> cg01615818

<400> 6
gacgcccggg gggctggctg tcagcgcagc cgccatctta tggtccaagt ggactgaggc 60

cgcctgggcg aggagaactc agggcgcaaa ttgtggagct gactgcggcg aggtctgagt 120

cc 122

<210> 7
<211> 122
<212> DNA
<213> Artificial Sequence

<220>
<223> cg09255157

<400> 7
tggaaaagaa ttcaagtcac caaatcttaa gcatttattt agtgtttgtg ctgtgtcaag 60

cgtagttctg ggggctaagt atacaaaagt aaataagact tagattctgc cctaaggaac 120

tc 122

<210> 8
<211> 122
<212> DNA
<213> Artificial Sequence

<220>
<223> cg14234990

<400> 8
ccttcttgac aatgatgaca atagcactgc cttccctttg ttacacactg tgtgtcaggc 60

cgtgtactga ccacttctct tgcattctct ctccttctct tcacaagagc cccatgaagt 120

gg 122

<210> 9
<211> 122
<212> DNA
<213> Artificial Sequence

<220>
<223> cg04850148

<400> 9
ggggcccaca gctaaatcca gtgagtggaa gttacaggga gtctgcttcc agtgctgctc 60

cgggaaggat cccatccacc agagctgccc cacatggacc atggtcaggc agaggaagat 120

gc 122

<210> 10
<211> 122
<212> DNA
<213> Artificial Sequence

<220>
<223> cg00356897

<400> 10
tagccaaggt caagaactaa cctggggctg agtcagcgtc tctacccact taaataacag 60

cgtaaagatc tttcactaaa ttcgttatgt ggtctgtctg gatgtaaacc tatatatttc 120

ct 122

<210> 11
<211> 122
<212> DNA
<213> Artificial Sequence

<220>
<223> cg01847718

<400> 11
tcacgtctgg tcatcactct agcttcagac tcaaaagtga acgattggct ggctgtgagc      60

cgcaggaccc tgcggggaga ccagggtggt ctctacactg ttgggtagct agctggaggc     120

aa                                                                    122


<210> 12
<211> 122
<212> DNA
<213> Artificial Sequence


<220>
<223> cg01257975

<400> 12
ccctccctgg ctgcccccgg ggggcagaga gttcggaggt gaccctgcga ccccagttgg      60

cgcgatggcc gcatgggcgg agaccggcaa ccctgcgcat cgctgacccc cggcgctccg     120

tc                                                                    122


<210> 13
<211> 122
<212> DNA
<213> Artificial Sequence


<220>
<223> cg09163005

<400> 13
aacctacctg agtagtacgg tgtattatat gagaacatcc acttagcata gcaacgtcta      60

cggacaaccc ctgtagctct tcttctggat tgttttctgt ttccaaccat gttttcttat     120

tt                                                                    122


<210> 14
<211> 122
<212> DNA
<213> Artificial Sequence


<220>
<223> cg12806797

<400> 14
cgggggacct ccacgtcttc gcagaggacc cgatccgctg atctgtggcg ccctggcttg      60

cggggagcaa gccaaggcca agactttggc ctctccaata aaacctagtg gctctttgca     120

gg                                                                    122


<210> 15

<211> 122
<212> DNA
<213> Artificial Sequence

<220>
<223> cg05014687

<400> 15
acagcaccca gtccccccgc tccgccacgg cacccagtcc cctcagggca cccggttccc 60

cgcctccgcc aaggcacccg gttccccgcc tccgccaagg caaccggtcc cccccccccc 120

cg 122

<210> 16
<211> 122
<212> DNA
<213> Artificial Sequence

<220>
<223> cg07112131

<400> 16
cggaccctgt cctgggcagg cagaattccg cggacaggct gttgtttcgc ctaagcagca 60

cgggctgcgg agacagaaac ctacctgagt agtacggtgt attatatgag aacatccact 120

ta 122

<210> 17
<211> 122
<212> DNA
<213> Artificial Sequence

<220>
<223> cg16322792

<400> 17
ttccccactt ccttcccctg ggtcagtccc aggatatcta ccccccacag gctccccaga 60

cggcagcctc ccgcggaccc agcccctaac acaggtgcag cttctggtgc tgcgcgaggt 120

gc 122

<210> 18
<211> 122
<212> DNA
<213> Artificial Sequence

<220>
<223> cg09169455

<400> 18
cagcactgac ttccacacgt catagaagct tccacgtgct ccagcttcaa gtatgctcca 60

cgccccattc tccccttcta attttctatg gcaatgacct aatctaacat ttttgcaact 120

tg                                                                 122

<210> 19
<211> 122
<212> DNA
<213> Artificial Sequence

<220>
<223> cg24491749

<400> 19
caaaggataa tgaacaagtc agactctgtg ccctcaaaga acttagagtt ttaaacgact      60

cgaatgggga aagaaagtaa aaaatagggc aaaagacgcc gattttatca tgaataaaat     120

tt                                                                 122

<210> 20
<211> 122
<212> DNA
<213> Artificial Sequence

<220>
<223> cg03856973

<400> 20
cgggcatccc ggtccgaaga cccaggaact gctctggcct ggcctggggt ctacacctga      60

cgcctggcga gggttggctt cgggtctggt cccaccgggc ctgggaggga cgggcggctg     120

ga                                                                 122

<210> 21
<211> 122
<212> DNA
<213> Artificial Sequence

<220>
<223> cg20649823

<400> 21
tatctgcggc cggagctcca ggtctcgtcc tcactactct gtgtcttctg cttttagggg      60

cgcagcctct gtggccctgt gacctgcccc ctggaagcct agaaatggtg agagtgccgg     120

gt                                                                 122

<210> 22
<211> 122
<212> DNA
<213> Artificial Sequence

<220>
<223> cg23831005

<400> 22
gataatagcc tgctctatgc ctgcaccttg aaataatcct ctaaaaatat atgactccta 60

cgttttaaaa atctccaggg aaaaaatatt taccaactcc atgaggattt tattatgtat 120

ct 122

<210> 23
<211> 122
<212> DNA
<213> Artificial Sequence

<220>
<223> cg07068406

<400> 23
tttagaagcc aggggaagag tgtgtcaggg aggagggcac aaaagtggca aatgcttctg 60

cgtgttaaac atttccactg ggtttagctg caagaaccct atggttaagt atacagatgg 120

tt 122

<210> 24
<211> 122
<212> DNA
<213> Artificial Sequence

<220>
<223> cg05871254

<400> 24
attatttaca gccgaagagg acacttcggc tttttcggac cttattttgg gtctgggaag 60

cgggaggggt tgctggcaag aaagtgaatt cagttgagtc aatttgcaac gagcacgttt 120

cg 122

<210> 25
<211> 122
<212> DNA
<213> Artificial Sequence

<220>
<223> cg17758362

<400> 25
ccactggatg ttgcatggag tgttgcctcc agttgacttt gatagacaca ataaacacca 60

cgtgagagcc ccctcatgaa ggagggctga ggacttggag tccaattctc cacctcccct 120

ga 122

<210> 26
<211> 122
<212> DNA

<213> Artificial Sequence

<220>
<223> cg06524124

<400> 26
agactctgtg attgactggc atagagccca ttcaaatgag cttaagcaca gggaagtttt 60

cgagaagaat ataaggacat cttaaggaac cccagggcag gaagtacagc ccagcctcag 120

ga 122

<210> 27
<211> 122
<212> DNA
<213> Artificial Sequence

<220>
<223> cg26127836

<400> 27
ccgtcagcac aggaagcatt cacgtctggt catcactcta gcttcagact caaaagtgaa 60

cgattggctg gctgtgagcc gcaggaccct gcggggagac cagggtggtc tctacactgt 120

tg 122

<210> 28
<211> 122
<212> DNA
<213> Artificial Sequence

<220>
<223> cg01249606

<400> 28
gcctttaaga agatcatgtt agccatcaaa tcaaggcaaa tgacataagc aagacgtgca 60

cgtcaccact agcaaaaggt aactcgggta aggcctggca aggcaaatga tcaccacatg 120

at 122

<210> 29
<211> 122
<212> DNA
<213> Artificial Sequence

<220>
<223> cg12754571

<400> 29
gagtcccggc ggctcggcgc ggtcttggtc tgggtcggtg gggttgggcc gggagacccg 60

cggccggcgg ggtccaggct gggggaggcc gccgctgcca ccaggtcccc gaggtagggt 120

ag 122

<210> 30
<211> 122
<212> DNA
<213> Artificial Sequence

<220>
<223> cg19736195

<400> 30
cctcgagggg ctaggtatga gtctgcaatt atgataggcg gccatcaggg ggcatttcag      60

cgccttggtc gtgtggccaa aggatcaggt ttaccataat cagagaataa ggtaattttt     120

ac                                                                    122

<210> 31
<211> 122
<212> DNA
<213> Artificial Sequence

<220>
<223> cg23133460

<400> 31
cagttttcag agtcttctat aaggtcttat accagccgag aagcagcaga accatctgtc      60

cgtgtaccga ggacggatct cagcagctct ctgttcagtg gctcctgcct ttgtagtgga     120

tt                                                                    122

<210> 32
<211> 122
<212> DNA
<213> Artificial Sequence

<220>
<223> cg23043514

<400> 32
acagcagcac aagtggacta agacgctgtc aacacttgtt aatttctgtt gttatgccat      60

cgtagtggtt gtaaactgat atcatgttgt gattttgatt tgcatttccc tgatgattag     120

tg                                                                    122

<210> 33
<211> 122
<212> DNA
<213> Artificial Sequence

<220>
<223> cg06277607

<400> 33

acgctttgca cgtcttagtg tgctcgactt ctctgcacta aacaccatgc cacactgccc 60

cgctcccccg gcatctctct ggcgctttct gctcagcctc tctgtggact cctcttcctc 120

tg 122

<210> 34
<211> 122
<212> DNA
<213> Artificial Sequence

<220>
<223> cg12418129

<400> 34
ttttaaataa ggcctttaaa atagtatcag aactgcttct ttttgtctgt ctcctgaacc 60

cgtatttccc agacattgtc agtgcttttg tgcttgtgtt tttgcccgag tggggtgcag 120

gg 122

<210> 35
<211> 122
<212> DNA
<213> Artificial Sequence

<220>
<223> cg10564845

<400> 35
tctcaatgct cagataagga caccagagtt cagccagaag cctggcaggg agctgtccac 60

cgccgtggtg ctgattgagg aggtggcttc ttacaaatct atcagcccac tcttttctta 120

aa 122

<210> 36
<211> 122
<212> DNA
<213> Artificial Sequence

<220>
<223> cg13959831

<400> 36
ctataattta cttagacaag caagccattt gccttgaggc tagacatgta aacaaacaca 60

cgtctaaagg cttggtctga gttggatttt taataatttc ttctccctta cacacacaca 120

ta 122

<210> 37
<211> 122
<212> DNA
<213> Artificial Sequence

<220>
<223> cg21593676

<400> 37
aaggaagcag ttgattgacc tgagagctcg ctgctaaaaa gcagtgcaag tggccatgcg      60

cggtggctca cgcctgtaat cccagcactt tgggaggctg aggcgggcgc atcacgaggt     120

ca                                                                    122


<210> 38
<211> 122
<212> DNA
<213> Artificial Sequence


<220>
<223> cg02847897

<400> 38
ctaaggaagc agttgattga cctgagagct cgctgctaaa aagcagtgca agtggccatg      60

cgcggtggct cacgcctgta atcccagcac tttgggaggc tgaggcgggc gcatcacgag     120

gt                                                                    122


<210> 39
<211> 122
<212> DNA
<213> Artificial Sequence


<220>
<223> cg13679303

<400> 39
ctgagtgctg ggtggtcccg cggaaagtgc ctggggctct gcagggctct caagagaccg      60

cgtgcggcct gggggcctgg ctctgggggc tgccatccgg gatcagaaaa tgtcccagag     120

ga                                                                    122


<210> 40
<211> 122
<212> DNA
<213> Artificial Sequence


<220>
<223> cg14598338

<400> 40
agcggtcggg accctcgggc aatgagagca aggtagagtc catccgagag gacacgtccg      60

cggtttgctg caaaaagccc cctttggctg ctgcgtggag ggaggctccg gagagcgtgg     120

aa                                                                    122

<210> 41
<211> 122
<212> DNA
<213> Artificial Sequence

<220>
<223> cg06031268

<400> 41
cgctgtcgag tgattgttct tgagaagctt ccagtagagg caatggtgac tattttaatg 60

cgagcgatca actccctggg aatccacgtc ctagactcta gccgtcccac tgaccctctg 120

ag 122

<210> 42
<211> 122
<212> DNA
<213> Artificial Sequence

<220>
<223> cg20603222

<400> 42
cctcctcact gctcttgcct ccgggcatgg gaacgatgcc gtctgctcgt ctgcctgaga 60

cgctttctac tccactccga gagtaggagg tcagccgggg ccaaatgaca tggtggacat 120

ca 122

<210> 43
<211> 122
<212> DNA
<213> Artificial Sequence

<220>
<223> cg26224354

<400> 43
ctcccgcagc cggcctcctc actgctcttg cctccgggca tgggaacgat gccgtctgct 60

cgtctgcctg agacgctttc tactccactc cgagagtagg aggtcagccg gggccaaatg 120

ac 122

<210> 44
<211> 122
<212> DNA
<213> Artificial Sequence

<220>
<223> cg06378142

<400> 44
taagaaccag tatgttacag ataatggcag tagctcacag tcacggggca tctcactaca 60

cgacaggctg cctcctgaga agctgatgga tgttaacttg tgtaattttc acccatccta 120

gt 122

<210> 45
<211> 122
<212> DNA
<213> Artificial Sequence

<220>
<223> cg06406458

<400> 45
ccatgggtct ctgtgcccct ttgtctcctg tggtcttggt cgtcccgtgc cctccaggct 60

cgcgttggac gacgccattc gacacaagcc gctgaacatg tcatcccgtt tttcacccag 120

gg 122

<210> 46
<211> 122
<212> DNA
<213> Artificial Sequence

<220>
<223> cg05622025

<400> 46
aactttctta aggtctcata accactagat ggcagagcct ggattcaaat tcagaattat 60

cgttcttcaa agcctgcata tttattttaa ataggaagct gacaaagcaa atggaaaaag 120

tc 122

<210> 47
<211> 122
<212> DNA
<213> Artificial Sequence

<220>
<223> cg09715000

<400> 47
taacttggcc aaggttgctc agcaaataag agacaaatca gaatttacac ccaggcagtc 60

cggctcagaa atctagggtc ttcaccatga cacaatgcca ccattccaat atctgccttt 120

at 122

<210> 48
<211> 122
<212> DNA
<213> Artificial Sequence

<220>

<223> cg26913836

<400> 48
gcgcacatgc ctgagcgccc tctgcatatg agtgtgcaca cacgcatggg tgtgtttaca      60

cgtctgtttg cgtaatttac cgggtttgca tgtgcatcaa gacatgtatg tgtgtgctaa     120

tg                                                                    122


<210> 49
<211> 122
<212> DNA
<213> Artificial Sequence


<220>
<223> cg15488109

<400> 49
acagagccca aagccgccca cttccccgga tacctgtgct tcagaccccc gtgggcacca      60

cgggagggcc tctgatcagg gcctgatctt ctcagactgc cctgcaaccc aagtgggact     120

cg                                                                    122


<210> 50
<211> 122
<212> DNA
<213> Artificial Sequence


<220>
<223> cg25873494

<400> 50
agaacattag cctctataac cctattataa ttctaggaaa actcgtcagt gtatgaccta      60

cggtttcata tgtgttgacc ttggtaaata caggaagggg gttagtcata agtcagtgtg     120

tt                                                                    122


<210> 51
<211> 122
<212> DNA
<213> Artificial Sequence


<220>
<223> cg25123302

<400> 51
tgattttaaa aatatttaca attgagatga taaaacaact catttgtcat tggaaaattg      60

cgctacacca cacaactttg tgcttcaggt tttcggatgg aggtggctgc agaccttagg     120

ta                                                                    122


<210> 52
<211> 122

<212> DNA
<213> Artificial Sequence

<220>
<223> cg01379846

<400> 52
cgatcgtgtc gattttgcgg ggccggctgc cgcctaggcg gggacgccca cggcccaggt 60

cggcctcagt ttccaggtag tagacgttga aggtctcctt gcaggtaccc gcggcgccag 120

gg 122

<210> 53
<211> 122
<212> DNA
<213> Artificial Sequence

<220>
<223> cg08944241

<400> 53
aagtcactgc tgtagactga atggctgagt gccctcccaa ggtcacatgt tcaaacctaa 60

cgcccagtgt gatggcgttt ggagatacgt gctcatgaat gaggttagtg cccttataaa 120

ag 122

<210> 54
<211> 122
<212> DNA
<213> Artificial Sequence

<220>
<223> cg04377463

<400> 54
caaatacgta cccatcagat ttacctgttt aacttaaact ataattactg ggcaatcaga 60

cggctgacaa gtgtaggtac taattatcca aatgagactc cggctgccca gaggtaccta 120

gt 122

<210> 55
<211> 122
<212> DNA
<213> Artificial Sequence

<220>
<223> cg03299990

<400> 55
aagctgagtg ggatcattcc tcttaagaat tcttacagca gccagataag aggccaattc 60

cgtcagggag agagagaatg cgaggtggag ggccctggtg ggaagatggg ctgttgttgc 120

cg                                                                 122

<210> 56
<211> 122
<212> DNA
<213> Artificial Sequence

<220>
<223> cg00683245

<400> 56
gccacctgag agcagcacga catgtttgaa acatgtgtct gggtttctct tctccctaaa      60

cgcccgacct ttgttgactt tcaagtagca cggctgctgc tgaataataa caaagaacaa     120

ac                                                                 122

<210> 57
<211> 122
<212> DNA
<213> Artificial Sequence

<220>
<223> cg12793466

<400> 57
caatttccag atctaacttc tatttactta tgtgttccat ttttacccac ttttgattac      60

cgtgttctca ctgcctgttc tgtggtagcc tcgacaactt ttaaatggtc tttaatcacc     120

aa                                                                 122

<210> 58
<211> 122
<212> DNA
<213> Artificial Sequence

<220>
<223> cg05894480

<400> 58
gaaggcaggc tggtccccag ctcttcagag tggagctggg aagagctgca gctgccccaa      60

cgctgggaag tcaccagtcc ttagggttgc tacgtttagc agatgaaaat tcaggatggg     120

cc                                                                 122